# EUROPÄISCHE PATENTANMELDUNG

(11) **EP 3 086 325 A1**
(43) Veröffentlichungstag der Anmeldung: **26.10.2016**
(21) Anmeldenummer: 16165260.7
(22) Anmeldetag: 14.04.2016
(51) Int. Cl.: G21K 1/093, G21K 5/04, A61N 5/00

(54) **STRAHLFÜHRUNGSSYSTEM, TEILCHENSTRAHL-THERAPIEANLAGE UND VERFAHREN**

(30) Priorität: 23.04.2015 DE 102015106246
(71) Anmelder: CRYOELECTRA GMBH, 42287 Wuppertal (DE)
(72) Erfinder: DREES, Prof. DR., Jürgen, 42287 Wuppertal (DE); PIEL, Prof. DR., Helmut, 42287 Wuppertal (DE)
(74) Vertreter: Cohausz & Florack

(57) **Zusammenfassung**

Die Erfindung betrifft unter anderem ein Strahlführungssystem (2) zur Führung eines Strahls geladener Teilchen (6) mit einer magnetischen Strahlablenkeinheit (30a, 30b, 30c, 30d), wobei die magnetische Strahlablenkeinheit (30a, 30b, 30c, 30d) eine Eintrittsseite (32a, 32b, 32c, 32d) zum Eintreten des Strahls geladener Teilchen unter einer Eintrittsrichtung in die magnetische Strahlablenkeinheit (30a, 30b, 30c, 30d) aufweist, wobei die magnetische Strahlablenkeinheit (30a, 30b, 30c, 30d) eine Austrittsseite (34a, 34b, 34c, 34d) zum Austreten des Strahls geladener Teilchen unter einer Austrittrichtung aus der magnetische Strahlablenkeinheit (30a, 30b, 30c, 30d) aufweist. Gemäß einem weiteren Aspekt betrifft die Erfindung eine vorteilhafte Teilchenstrahl-Therapieanlage (1). Die Aufgabe, verbesserte Strahleigenschaften bereitzustellen und gleichzeitig den Platzbedarf zu reduzieren, wird dadurch gelöst, dass die Eintrittsseite (32a, 32b, 32c, 32d) der magnetischen Strahlablenkeinheit (30a, 30b, 30c, 30d) zumindest abschnittsweise im Wesentlichen parallel zu der Austrittsseite (34a, 34b, 34c, 34d) der magnetischen Strahlablenkeinheit (30a, 30b, 30c, 30d) ausgebildet ist.

## Beschreibung

Die Erfindung betrifft gemäß einem Aspekt ein Strahlführungssystem zur Führung eines Strahls geladener Teilchen mit einer magnetischen Strahlablenkeinheit, wobei die magnetische Strahlablenkeinheit eine Eintrittsseite zum Eintreten des Strahls geladener Teilchen unter einer Eintrittsrichtung in die magnetische Strahlablenkeinheit aufweist, und wobei die magnetische Strahlablenkeinheit eine Austrittsseite zum Austreten des Strahls geladener Teilchen unter einer Austrittrichtung aus der magnetische Strahlablenkeinheit aufweist. Gemäß einem weiteren Aspekt betrifft die Erfindung eine vorteilhafte Teilchenstrahl-Therapieanlage. Die Erfindung betrifft zudem gemäß einem weiteren Aspekt ein vorteilhaftes Verfahren.

Eingangs genannte Strahlführungssysteme werden im Stand der Technik beispielsweise in Teilchenstrahl-Therapiesystemen zur Strahlentherapie mit geladenen Teilchen, beispielsweise Ionen in Form von Protonen, verwendet. Dies bietet gegenüber der bisher üblichen Strahlentherapie mit Photonen wesentliche Vorteile für Patienten mit bestimmten Krebserkrankungen. Insbesondere ist eine Bestrahlung mit Ionen, insbesondere Protonen, vorteilhaft, da diese erst am Ende ihres Laufwegs im zu bestrahlenden Gewebe, im so genannten Bragg-Peak, ihre maximale Ionisationsstärke, und damit einhergehend ihre größte Zerstörungskraft, beispielsweise für Tumorzellen, aufweisen. Auf diese Weise kann die Beeinträchtigung von gesundem Gewebe, welches dem zu behandelnden Gewebe im Laufweg des Strahls vorgelagert ist und von dem Strahl durchlaufen wird, reduziert werden. Zudem kann die Beeinträchtigung von gesundem Gewebe, welches dem Strahl nachgelagert ist, fast vollständig vermieden werden.

Die erfolgreiche Behandlung von Tumoren mit einem Teilchenstrahl, beispielsweise mittels des Raster-Scan-Therapieverfahrens, erfordert allerdings einen Teilchenstrahl mit präzisen Strahleigenschaften, insbesondere der Strahlposition oder des Strahlimpulses, sodass am Behandlungsort (am sogenannten Isozentrum) eine präzise Behandlung des Gewebes erfolgen kann und Behandlungsungenauigkeiten vermieden werden, sodass möglichste keine unerwünschte Bestrahlung von gesundem Gewebe (etwa angrenzende Organe) erfolgt.

Die Teilchenstrahl-Therapiesysteme aus dem Stand der Technik, das heißt die Gesamtsysteme und insbesondere die Strahlführungssysteme, nehmen allerdings vergleichsweise viel Platz ein. Zudem sind die Strahleigenschaften und die erreichbare Transmission des Teilchenstrahls bis zum Behandlungsort verbesserungsbedürftig.

Aus der WO 2009/106603 A1 ist beispielsweise ein Teilchenstrahl-Therapiesystem bekannt, wobei ein Teilchenstrahl erzeugt und über ein Strahlführungssystem einem von mehreren Behandlungsräumen zugeführt wird. Es wird vorgeschlagen, zur Reduzierung des betrieblichen Aufwandes unterschiedliche Strahlführungssysteme vorzusehen, welche an bestimmte Strahleigenschaften angepasst sind. Dies kann durch die Verwendung einer einzigen Strahlerzeugungseinheit für mehrere Behandlungsorte zwar eine Platzersparnis bringen. Allerdings ist dies nur möglich, sofern mehrere Behandlungsorte vorgesehen werden und ohnehin viel Platz vorhanden ist. Zudem benötigen das Strahlführungssystem und die darin vorgesehenen Weichen weiterhin vergleichsweise viel Raum.

Aus der EP 2 268 359 B1 ist ebenfalls ein Teilchenstrahl-Therapiesystem bekannt, wobei zur Gewährleistung einer präzisen Strahlführung vorgeschlagen wird, mittels eines Strahlpositionsmonitors die Strahleigenschaften während der Behandlung im Halo-Bereich des Teilchenstrahls vorzunehmen. Dies ermöglicht zwar eine verbesserte Überprüfung der Strahleigenschaften. Allerdings sind die Strahleigenschaften weiterhin vorrichtungstechnisch begrenzt, sodass weiterhin das Bedürfnis besteht, die Strahleigenschaften zu verbessern. Zudem benötigt auch dieses System vergleichsweise viel Raum.

Hiervon ausgehend ist es Aufgabe der vorliegenden Erfindung, ein gattungsgemäßes Strahlführungssystem anzugeben, welches bei reduziertem Platzbedarf vergleichbare oder sogar verbesserte Strahleigenschaften bereitstellen kann. Weiterhin liegt der vorliegenden Erfindung die Aufgabe zugrunde, eine vorteilhafte Teilchenstrahl-Therapieanlage und ein vorteilhaftes Verfahren vorzuschlagen.

Gemäß einem Aspekt der vorliegenden Erfindung wird die Aufgabe bei einem gattungsgemäßen Strahlführungssystem mit einer magnetischen Strahlablenkeinheit dadurch gelöst, dass die Eintrittsseite der magnetischen Strahlablenkeinheit zumindest abschnittsweise im Wesentlichen parallel zu der Austrittsseite der magnetischen Strahlablenkeinheit ausgebildet ist.

Bisher werden im Stand der Technik magnetische Strahlablenkeinheiten verwendet, deren Eintrittsseite und Austrittsseite abgewinkelt zueinander verlaufen, da die Eintrittsseite und/oder die Austrittsseite in der Regel senkrecht zum Strahl ausgerichtete wird. Wird also beispielsweise eine magnetische Strahlablenkeinheit zur Ablenkung des Strahls geladener Teilchen um 45° vorgesehen, schließen auch die Eintrittsseite und die Austrittsseite der magnetischen Strahlablenkeinheit einen Winkel von 45° ein.

Die Erfindung wendet sich hiervon ab und schlägt eine Strahlablenkeinheit vor, deren Eintrittsseite zumindest abschnittsweise im Wesentlichen parallel zu der Austrittsseite ausgebildet ist. Es hat sich gezeigt, dass, wenn ein Strahl geladener Teilchen von einer Eintrittsrichtung in eine von der Eintrittsrichtung abweichende Austrittsrichtung abgelenkt werden soll, durch die erfindungsgemäße Ausbildung der Strahlablenkeinheit zum einen verbesserte Strahleigenschaften und gleichzeitig kompaktere Strahlführungssysteme bereitgestellt werden können, welche im Stand der Technik bisher nicht erreichbar waren. Dies wird unter anderem darauf zurückgeführt, dass derartige magnetische Strahlablenkeinheiten nicht nur ein Ablenken des Strahls geladener Teilchen, sondern zudem auch eine Fokussierung des Strahls geladener Teilchen ähnlich einer Strahlformungseinheit erreichen können. Denn die magnetoptischen Eigenschaften einer magnetischen Strahlablenkeinheit (zum Beispiel eines Dipolmagneten) werden wesentlich durch den Winkel des eintreffenden Strahls geladener Teilchen gegenüber der Eintritts- und Austrittskante bestimmt. Dadurch sind im gesamten Strahlführungssystem weniger Strahlformungseinheiten notwendig. Zudem kann eine erhöhte Transmission des Strahls geladener Teilchen durch das Strahlführungssystem erreicht werden. Dies führt dazu, dass insgesamt eine kompaktere Teilchenstrahl-Therapieanlage mit verbesserten Strahleigenschaften bereitgestellt werden kann. Zusätzlich ist auch die Fertigung von Strahlablenkeinheiten, bei denen die Eintrittsseite zumindest abschnittsweise im Wesentlichen parallel zu der Austrittsseite ausgebildet ist, einfacher durchzuführen, was zusätzlich den Herstellungsaufwand reduziert.

Beispielsweise ist die Eintrittsseite und/oder die Austrittsseite der magnetischen Strahlablenkeinheit zumindest abschnittsweise flächig ausgebildet. In dem Fall sind insbesondere die flächigen Abschnitte der Eintrittsseite und der Austrittsseite zumindest abschnittsweise im Wesentlichen parallel zueinander ausgebildet. Die Eintrittsseite kann insbesondere eine Stirnfläche, beispielsweise eine Vorderseite der Strahlablenkeinheit sein, welche beispielsweise eine Eintrittsöffnung zum Eintreten des Strahls geladener Teilchen aufweist. Die Austrittsseite kann insbesondere eine weitere Stirnfläche, beispielsweise eine der Eintrittsseite gegenüberliegende Stirnfläche, beispielsweise eine Rückseite der Strahlablenkeinheit sein, welche beispielsweise eine Austrittsöffnung zum Austreten des Strahls geladener Teilchen aufweist.

Bevorzugt sind die gesamte Eintrittsseite und die gesamte Austrittsseite im Wesentlich parallel zu einander. Unter im Wesentlichen parallel wird verstanden, dass die Eintrittsseite und die Austrittsseite beispielsweise einen Winkel von weniger als 5°, vorzugsweise einen Winkel von weniger als 3°, besonders bevorzugt weniger als 1° einschließen.

Die magnetische Strahlablenkeinheit ist zur Ablenkung des Strahls geladener Teilchen beispielsweise derart in dem Strahlführungssystem vorgesehen, dass der Strahl von der Eintrittsrichtung in die gewünschte Austrittsrichtung abgelenkt wird.

Die magnetische Strahleinheit ist bevorzugt zu einer zeitlich konstanten Ablenkung des Strahls geladener Teilchen ausgebildet. Das heißt, dass der Strahl geladener Teilchen beispielsweise dauerhaft um 45° abgelenkt wird.

In dem Strahlführungssystem kann auch mehr als eine Strahlablenkeinheit vorgesehen sein. Beispielsweise sind zwei, drei oder vier oder mehr Strahlablenkeinheiten vorgesehen. Ist in dem Strahlführungssystem mehr als eine Strahlablenkeinheit vorgesehen, können beispielsweise alle oder nur einige (beispielsweise eine, zwei, drei oder vier) der Strahlablenkeinheiten erfindungsgemäß ausgestaltet sein. Insofern ist die Formulierung eine/die Strahlablenkeinheit als wenigstens eine/die wenigstens eine Strahlablenkeinheit zu verstehen.

Der Strahl von Teilchen ist bevorzugt ein Ionenstrahl, insbesondere ein Protonenstrahl. Das Strahlführungssystem ist beispielsweise dazu ausgelegt, den Strahl geladener Teilchen mit Energien von 60 bis 210 MeV gezielt zu führen. 1 MeV entspricht ungefähr 1,6 x 10⁻¹³ Joule. Dabei wird unter der Energie der Teilchen insbesondere die mittlere oder maximale kinetische Energie der Teilchen verstanden.

Die Strahlablenkeinheit kann beispielsweise derart ansteuerbar sein, dass eine gewünschte Ablenkung beispielsweise für unterschiedliche Energien der geladenen Teilchen des Strahls der geladenen Teilchen möglich ist.

Gemäß einer bevorzugten Ausgestaltung des erfindungsgemäßen Strahlführungssystems ist die magnetische Strahlablenkeinheit derart zur Ablenkung des Strahls geladener Teilchen in dem Strahlführungssystem vorgesehen, dass die Eintrittsseite schräg zur Eintrittsrichtung des Strahls geladener Teilchen und/oder die Austrittsseite schräg zur Austrittsrichtung des Strahls geladener Teilchen liegt.

Der Strahl geladener Teilchen kann in dem Strahlführungssystem insbesondere unter einer definierten Eintrittsrichtung in die Strahlablenkeinheit eintreten und unter einer definierten Austrittsrichtung aus der Strahlablenkeinheit austreten. Dadurch, dass die Eintrittsseite schräg zur Eintrittsrichtung des Strahls geladener Teilchen bzw. die Austrittsseite schräg zur Austrittsrichtung des Strahls geladener Teilchen liegt, kann die Strahlablenkeinheit insbesondere symmetrisch in dem Strahl geladener Teilchen vorgesehen werden, was die Strahleigenschaften weiterhin verbessert. Unter einer schrägen Anordnung wird dabei insbesondere verstanden, dass eine Seite insbesondere nicht senkrecht auf und/oder nicht parallel zu der Eintritts- oder Austrittsrichtung steht. Beispielsweise verlaufen die Eintritts- und/oder die Austrittsseite im Wesentlichen parallel zu der Mittelsenkrechten des Winkels, der zwischen der Eintrittsrichtung und der Austrittsrichtung des Strahls geladener Teilchen gebildet wird. Beispielsweise ist der Winkel zwischen der Eintrittsseite und der Eintrittsrichtung und zwischen der Austrittsseite und der Austrittsrichtung gleich.

Gemäß einer bevorzugten Ausgestaltung des erfindungsgemäßen Strahlführungssystems ist die magnetische Strahlablenkeinheit derart zur Ablenkung des Strahls geladener Teilchen in dem Strahlführungssystem vorgesehen, dass die Eintrittsrichtung und die Austrittsrichtung in einem Winkel von 30° bis 60°, vorzugsweise von 40° bis 50°, insbesondere im Wesentlichen 45°, zueinander stehen.

Der Strahl geladener Teilchen wird also beispielsweise um 30° bis 60°, vorzugsweise um 40° bis 50°, insbesondere um im Wesentlichen 45°, abgelenkt. Es hat sich gezeigt, dass eine Ablenkung des Strahls geladener Teilchen bei diesen Winkeln mit guten Strahleigenschaften erfolgen und gleichzeitig ein kompaktes Strahlführungssystem bereitgestellt werden kann.

Gemäß einer bevorzugten Ausgestaltung des erfindungsgemäßen Strahlführungssystems ist die magnetische Strahlablenkeinheit ein Dipolmagnet.

Durch die Ausgestaltung der magnetischen Strahlablenkeinheit als Dipolmagnet kann auf einfache Weise ein im Wesentlichen homogenes Magnetfeld zur Ablenkung des Strahls geladener Teilchen bereitgestellt werden. Zudem können Dipolmagnete vergleichsweise einfach gefertigt werden. Die Fertigung wird vorliegend insbesondere dadurch vereinfacht, dass die Eintrittsseite zumindest abschnittsweise im Wesentlichen parallel zu der Austrittsseite ausgebildet ist. Der Dipolmagnet ist beispielsweise ein Elektromagnet. Beispielsweise weist der Dipolmagnet einen Eisenkern, beispielsweise ein Eisenjoch, auf. Der Eisenkern kann beispielsweise Eisenplatten aufweisen. Beispielsweise ist der Eisenkern durch aufeinander gestapelte Eisenplatten hergestellt. Dies ermöglicht eine einfache Fertigung des Dipolmagneten. Der Dipolmagnet kann beispielsweise in Richtung des Strahls geladener Teilchen gesehen einen Abstand der im Wesentlichen parallelen Eintrittsseite und Austrittsseite von 0,5 bis 2 m aufweisen, bevorzugt etwa 1 m.

Es hat sich gezeigt, dass der Einsatz von Dipolmagneten in dem Strahlführungssystem, wobei die Eintrittsseite zumindest abschnittsweise im Wesentlichen parallel zu der Austrittsseite ausgebildet ist, gute Strahleigenschaften ermöglicht. Dies wird darauf zurückgeführt, dass die Dipolmagnete durch die erfindungsgemäße Ausgestaltung ähnlich wie Quadrupolmagnete bei der Fokussierung des Strahls geladener Teilchen helfen können.

Gemäß einer bevorzugten Ausgestaltung des erfindungsgemäßen Strahlführungssystems weist das Strahlführungssystem mehrere, insbesondere vier magnetische Strahlablenkeinheiten auf.

Sind mehrere magnetische Strahlablenkeinheiten vorgesehen, welche erfindungsgemäß ausgestaltet sind, kann ein komplexer Verlauf des Strahls geladener Teilchen in einem kompakten Strahlführungssystem erreicht werden. Wie bereits ausgeführt, ist zumindest ein Teil, vorzugsweise sind alle der mehreren magnetischen Strahlablenkeinheiten erfindungsgemäß ausgestaltet.

Bevorzugt sind die mehreren Strahlablenkeinheiten derart in dem Strahlführungssystem vorgesehen, dass der Strahl geladener Teilchen (zu einem bestimmten Zeitpunkt) im Wesentlichen in einer Ebene verläuft.

Beispielsweise ist das Strahlführungssystem derart eingerichtet, dass der Strahl geladener Teilchen durch die erste Strahlablenkeinheit von der ursprünglichen Achse des Strahls geladener Teilchen weggelenkt wird und schräg zu der ursprünglichen Achse verläuft. Die ursprüngliche Achse des Strahls geladener Teilchen ist beispielsweise die Achse, entlang der sich der Strahl geladener Teilchen vor der ersten Strahlablenkeinheit bewegt. Beispielsweise ist das Strahlführungssystem derart eingerichtet, dass der Strahl geladener Teilchen durch die weiteren magnetischen Strahlablenkeinheiten (beispielsweise eine zweite, dritte und vierte magnetische Strahlablenkeinheit) wieder zu der ursprünglichen Achse des Strahls geladener Teilchen hinlenkt wird. Beispielsweise ist das Strahlführungssystem derart eingerichtet, dass der Strahl geladener Teilchen nach der zweiten Strahlablenkeinheit parallel zu der ursprünglichen Achse des Strahls geladener Teilchen verläuft. Beispielsweise ist das Strahlführungssystem derart eingerichtet, dass der Strahl geladener Teilchen nach der letzten (beispielsweise der vierten) Strahlablenkeinheit quer, insbesondere im Wesentlichen senkrecht, zu der ursprünglichen Achse des Strahls geladener Teilchen verläuft und vorzugsweise die ursprüngliche Achse kreuzt.

Vorzugsweise ist zumindest ein Teil der mehreren magnetischen Strahlablenkeinheiten identisch ausgebildet. Hierdurch kann der Fertigungsaufwand für die magnetischen Strahlablenkeinheiten reduziert werden. Beispielsweise sind alle bis auf eine magnetische Strahlablenkeinheit (beispielsweise die letzte magnetische Strahlablenkeinheit vor dem Behandlungsort) identisch ausgebildet. Beispielsweise ist die vierte Strahlablenkeinheit abweichend ausgebildet. Durch eine abweichende Ausbildung insbesondere der letzten magnetischen Strahlablenkeinheit kann eine Anpassung an die Bedingungen im Strahlverlauf erfolgen. Beispielsweise weist die vierte Strahlablenkeinheit eine im Vergleich zu den anderen magnetischen Strahlablenkeinheiten vergrößerte Eintrittsöffnung und/oder Austrittsöffnung auf, um eine Bestrahlung eines ausreichenden Volumens am Behandlungsort zur Verfügung zu stellen.

Beispielsweise sind die in Richtung des Strahls geladener Teilchen gesehen zweite und dritte magnetische Strahlablenkeinheit 0,5 m bis 2 m, vorzugsweise 1 m bis 1,5 m voneinander entfernt. Beispielsweise ist die in Richtung des Strahls geladener Teilchen gesehen letzte magnetische Strahlablenkeinheit höchstens 1,5 m, vorzugsweise höchstens 1 m vom Behandlungsort entfernt. Unter dem Abstand von zwei magnetischen Strahlablenkeinheiten wird insbesondere der Abstand von dem Austrittspunkt des Strahls geladener Teilchen aus der einen magnetischen Strahlablenkeinheit bis zum Eintrittspunkt des Strahls geladener Teilchen in die andere magnetische Strahlablenkeinheit verstanden.

Der Strahl geladener Teilchen kann mit dem Strahlführungssystem beispielsweise auf einer Strecke von weniger als 10 m, insbesondere weniger als 8 m von der Richtung entlang der ursprünglichen Achse des Strahl geladener Teilchen in eine Richtung senkrecht hierzu und die ursprüngliche Achse kreuzend abgelenkt werden.

Gemäß einer bevorzugten Ausgestaltung des erfindungsgemäßen Strahlführungssystems weist das Strahlführungssystem wenigstens eine magnetische Strahlformungseinheit auf, insbesondere mehrere magnetische Strahlformungseinheiten, vorzugsweise in Form von einem oder mehreren Quadrupolmagneten.

Durch das Vorsehen von magnetischen Strahlformungseinheiten, insbesondere in Form von Quadrupolmagneten, kann die Strahleigenschaft weiter verbessert werden und der Strahl geladener Teilchen effizient fokussiert werden. Es hat sich gezeigt, dass in dem Strahlführungssystem vergleichsweise wenige Strahlformungseinheiten notwendig sind, um gute Strahleigenschaften zu erzielen, was ein kompaktes Strahlführungssystem erlaubt. Grundsätzlich können aber auch andere Strahlformungseinheiten vorgesehen sein. Beispielsweise können Strahlformungseinheiten in Form von Sextupolmagnete vorgesehen sein.

Quadrupolmagnete können aufgrund ihres Aufbaus einen Strahl geladener Teilchen nur in einer Richtung transversal zum Strahl geladener Teilchen fokussieren. Insofern ist es vorteilhaft mindestens zwei Quadrupolmagnete vorzusehen, um eine Strahlformung in beide Richtungen (d.h. in der Ebene) transversal zum Strahl geladener Teilchen zu erreichen.

Beispielsweise sind mindestens fünf und/oder höchstens zehn, vorzugsweise sieben Strahlformungseinheiten in dem Strahlführungssystem vorgesehen. Beispielsweise sind mindestens vier und/oder höchstens sechs, vorzugsweise fünf Strahlformungseinheiten zwischen der (in Richtung des Strahls geladener Teilchen gesehen) ersten und der zweiten magnetischen Strahlablenkeinheit vorgesehen. Beispielsweise sind zwei Strahlformungseinheiten zwischen der zweiten und der dritten magnetischen Strahlablenkeinheit vorgesehen. Es können jedoch auch weitere Strahlformungseinheiten vorgesehen sein. Beispielsweise sind die Strahlformungseinheiten zumindest teilweise, vorzugsweise alle gleich dimensioniert. Dies verringert den Herstellungsaufwand des Strahlführungssystems.

Gemäß einer weiteren bevorzugten Ausgestaltung des erfindungsgemäßen Strahlführungssystems weist das Strahlführungssystem in Richtung des Strahls geladener Teilchen gesehen vor der ersten magnetischen Strahlablenkeinheit eine Kollimatoreinheit auf.

Hierdurch können die Strahleigenschaften bei geringem zusätzlichem Platzbedarf verbessert werden. Durch die Kollimatoreinheit kann der Phasenraum des Strahls geladener Teilchen des hinter der Kollimatoreinheit weiter transportierten Strahls eingegrenzt werden. Der Phasenraum des Strahls geladener Teilchen wird insbesondere durch Ort und Impuls der Teilchen bestimmt. Je höher die Phasenraumdichte ist, desto schmaler ist im Regelfall das Strahlprofil und desto geringer ist im Regelfall die Divergenz des Teilchenstrahls. Ein Strahl mit hoher Phasenraumdichte erfordert einen geringeren Strahlführungsaufwand im Vergleich zu einem Teilchenstrahl mit geringerer Phasenraumdichte.

Beispielsweise ist die Kollimatoreinheit als Blende ausgebildet, beispielsweise als Materialblock mit einer oder mehreren Öffnungen. Beispielsweise weist die Kollimatoreinheit eine eckige, beispielsweise eine rechteckige Öffnung auf. Vorzugsweise lässt sich die Öffnung in beide Richtungen transversal zum Strahl geladener Teilchen verändern. Damit kann eine flexible Anpassung der Strahleigenschaften erfolgen.

Gemäß einer weiteren bevorzugten Ausgestaltung des erfindungsgemäßen Strahlführungssystems weist das Strahlführungssystem mindestens zwei magnetische Strahlablenkeinheiten und zwischen zwei der mindestens zwei magnetischen Strahlablenkeinheiten eine Kollimatoreinheit auf.

Es hat sich gezeigt, dass insbesondere zwischen zwei Strahlablenkeinheiten eine große Impulsdispersion herrschen kann. Durch das Vorsehen einer Kollimatoreinheit können die Strahleigenschaften bei geringem zusätzlichem Platzbedarf weiterhin verbessert werden. Es hat sich gezeigt, dass insbesondere zwischen der zweiten und dritten Strahlablenkeinheit eine vergleichsweise große Impulsdispersion herrscht, sodass durch das Vorsehen einer Kollimatoreinheit die Strahleigenschaften besonders verbessert werden können. Die Kollimatoreinheit kann daher insbesondere dazu verwendet werden, eine Impulsselektion für den Strahl geladener Teilchen am Behandlungsort zu erreichen. Wie bereits ausgeführt kann die Kollimatoreinheit beispielsweise als Blende ausgebildet sein, beispielsweise als Materialblock mit einer oder mehreren Öffnungen. Beispielsweise weist die Kollimatoreinheit eine eckige, beispielsweise eine rechteckige Öffnung auf. Vorzugsweise lässt sich die Öffnung in beide Richtungen transversal zum Strahl geladener Teilchen verändern. Damit kann eine flexible Anpassung der Strahleigenschaften erfolgen. Insbesondere kann eine Formgebung für den Strahlfleck am Behandlungsort erfolgen. Es kann zudem erreicht werden, dass die Verluste hinter dem Kollimator bis zum Behandlungsort kleiner 1% betragen, obwohl im Strahlführungssystem in Strahlrichtung gesehen weitere, beispielsweise noch zwei Strahlablenkeinheiten angeordnet sind.

Das Strahlführungssystem kann noch weitere hier nicht erwähnte Einheiten aufweisen. Beispielsweise kann ein Scanmagnet im Strahlführungssystem vorgesehen sein. Beispielsweise ist der Scanmagnet zwischen zwei Strahlablenkeinheiten vorgesehen. Vorzugsweise ist der Scanmagnet zwischen der letzten und der vorletzten (beispielsweise zwischen der dritten und der vierten) Strahlablenkeinheit vorgesehen, da der Scan-Magnet in dieser Position vorteilhaft einen großen Scan-Bereich am Behandlungsort ermöglicht.

Als weiteres Beispiel einer weiteren Einheit kann das Strahlführungssystem einen oder mehrere Strahlmonitore aufweisen. Durch Strahlmonitore können die Strahleigenschaften, beispielsweise die Strahlposition und der Teilchenimpuls des Strahls geladener Teilchen insbesondere an unterschiedlichen Stellen vermessen werden.

Gemäß einer weiteren bevorzugten Ausgestaltung des erfindungsgemäßen Strahlführungssystems ist das Strahlführungssystem zumindest abschnittsweise beweglich, insbesondere rotierbar ausgebildet.

Beispielsweise weist das Strahlführungssystem einen unbeweglichen und einen beweglichen Abschnitt auf. Beispielsweise weist das Strahlführungssystem zur Realisierung des beweglichen Abschnitts ein bewegliches Traggestell, eine sogenannte Gantry, auf. Das Traggestell kann in vorteilhafter Weise, insbesondere um eine horizontale Achse, um bis zu 360° rotierbar sein, um den Behandlungsort aus möglichst vielen Winkeln bestrahlen zu können. Die Rotationsachse des Traggestells fällt insbesondere mit der ursprünglichen Achse des Strahls geladener Teilchen (d.h. insbesondere mit der Achse des Strahls geladener Teilchen vor einem Ablenken durch die erste Strahlablenkeinheit) zusammen.

Der bewegliche Abschnitt (insbesondere die Gantry) kann beispielsweise alle oder einen Teil der zuvor erwähnten Elemente des Strahlführungssystems aufweisen, insbesondere einen oder mehrere (beispielsweise vier) Strahlablenkeinheiten, einen oder mehrere (beispielsweise sieben) Strahlformungseinheiten, einen oder mehrere (beispielsweise zwei) Kollimatoreinheiten, einen oder mehrere Scanmagnete und/oder einen oder mehrere Strahlmonitore.

Grundsätzlich ist es auch denkbar den beweglichen Abschnitt des Strahlführungssystems frei von Kollimatoreinheiten auszugestalten. Dadurch kann das Strahlführungssystem kompakter ausgebildet werden.

Beispielsweise kann der bewegliche Abschnitt des Strahlführungssystems derart ausgebildet werden, dass der Strahl geladener Teilchen derart zum Behandlungsort geführt wird, dass der Strahl geladener Teilchen maximal 3 m von der ursprünglichen Achse des Strahls geladener Teilchen entfernt verläuft.

Gemäß einer bevorzugten Ausführungsform des erfindungsgemäßen Strahlführungssystems weist das Strahlführungssystem eine Energiekorrektureinheit zur Einstellung der Energie der geladenen Teilchen des Strahls auf.

Durch die Energiekorrektureinheit wird eine flexible Anpassung der Energie der geladenen Teilchen des Strahls geladener Teilchen ermöglicht. Beispielsweise ist es möglich eine Strahlerzeugungseinheit (zum Beispiel ein Zyklotron) zu verwenden, welche geladene Teilchen mit im Wesentlichen konstanter Energie emittiert, ohne darauf verzichten zu müssen, die Energie der geladenen Teilchen des Strahls geladener Teilchen flexibel einstellen zu können. Beispielsweise ist die Energiekorrektureinheit zur Reduzierung der Energie der geladenen Teilchen des Strahls geladener Teilchen eingerichtet. Beispielsweise ist die Energiekorrektureinheit ein Degrader. Beispielsweise muss lediglich eine Strahlerzeugungseinheit, welche Teilchen mit ausreichender maximaler Energie emittiert, vorgesehen sein. Mittels der Energiekorrektureinheit kann die Energie der geladenen Teilchen des Strahls geladener Teilchen dann nach Bedarf reduziert werden.

Beispielsweise kann die Energie der geladenen Teilchen des Strahls geladener Teilchen reduziert werden, indem die (mittlere) kinetische Energie der geladenen Teilchen reduziert wird. Durch die Energiekorrektureinheit kann beispielsweise ein Abbremsen der geladenen Teilchen auf eine wählbare mittlere kinetische Energie erfolgen.

Es hat sich gezeigt, dass das Strahlführungssystem so kompakt gebaut werden kann, dass beispielsweise der Behandlungsort weniger als 10m, insbesondere weniger als 9 m vom Ende der Energiekorrektureinheit entfernt positioniert werden kann.

Beispielsweise kann eine Strahlerzeugungseinheit verwendet werden, welche geladene Teilchen mit einer kinetischen Energie von mehr als 200 MeV emittiert, beispielsweise zwischen 200 und 300 MeV, insbesondere zwischen 210 und 250 MeV. Die Energiekorrektureinheit kann beispielsweise dergestalt sein, dass die kinetische Energie der geladenen Teilchen beispielsweise auf unter 200 MeV oder auf unter 100 MeV reduziert werden kann.

Bevorzugt durchläuft der Strahl geladener Teilchen die Energiekorrektureinheit zumindest teilweise in einem Vakuum (beispielsweise mindestens ein Feinvakuum).

Die Energiekorrektureinheit ist vorzugsweise im unbeweglichen Abschnitt (insbesondere nicht im beweglichen Traggestell) des Strahlführungssystems vorgesehen.

Gemäß einer bevorzugten Ausführungsform des erfindungsgemäßen Strahlführungssystems weist die Energiekorrektureinheit mindestens ein quer zum Strahl geladener Teilchen verschiebbares blockförmiges Energiekorrekturelement und mindestens ein quer zum Strahl geladener Teilchen verschiebbares keilförmiges Energiekorrekturelement auf.

Ein blockförmiges Energiekorrekturelement umfasst beispielswiese eine Eintrittsseite und eine Austrittsseite für den Strahl geladener Teilchen, welche im Wesentlichen parallel verlaufen. Ein blockförmiges Energiekorrekturelement kann beispielsweise ein Quader sein.

Ein keilförmiges Energiekorrekturelement umfasst beispielsweise eine Eintrittsseite und eine Austrittsseite für den Strahl geladener Teilchen, welche schräg, das heißt nicht parallel, zueinander verlaufen.

Insbesondere durch die vorteilhafte Kombination mindestens eines blockförmigen und mindestens eines keilförmigen Energiekorrekturelements kann eine kompakte und präzise Reduzierung der Energie der geladenen Teilchen des Strahls geladener Teilchen erreicht werden. Insbesondere aufgrund der Kompaktheit (d.h. eine kurze von dem Strahl geladener Teilchen zu durchlaufende Strecke) kann durch eine solche Energiekorrektureinheit ein übermäßiges Aufweiten des Phasenraums des Strahls geladener Teilchen vermieden werden. Das blockförmige Energiekorrekturelement dient beispielsweise einer ersten groben Einstellung der Energie der geladenen Teilchen des Strahls geladener Teilchen. Beispielsweise kann die Energie der geladenen Teilchen des Strahls geladener Teilchen auf diskrete Werte eingestellt werden. Das keilförmige Energiekorrekturelement dient beispielsweise einer im Vergleich zur ersten Einstellung feineren Einstellung der Energie der geladenen Teilchen des Strahls geladener Teilchen. Beispielsweise kann die Energie der geladenen Teilchen des Strahls geladener Teilchen (beispielsweise in einem bestimmten Bereich) kontinuierlich eingestellt werden. Beispielsweise wird durch die Verschiebung des keilförmigen Energiekorrekturelements quer zum Strahl geladener Teilchen die in Richtung des Strahls geladener Teilchen gesehene Ausdehnung des keilförmigen Energiekorrekturelements im Bereich des Strahls geladener Teilchen verändert. Durch das Anordnen der blockförmigen Energiekorrekturelemente in Richtung des Strahls geladener Teilchen gesehen vor den keilförmigen Energiekorrekturelementen, können letztere entsprechend kürzer ausgebildet sein, da die Energie nur über einen kleineren Bereich eingestellt zu werden braucht.

Beispielsweise ist das mindestens eine blockförmige Energiekorrekturelement in eine Richtung (zum Beispiel in x-Richtung) quer zum Strahl geladener Teilchen verschiebbar. Beispielsweise ist das mindestens eine keilförmige Energiekorrekturelement in der gleichen und/oder in der dazu senkrechten transversalen Richtung (z.B. in x-Richtung und/oder in y-Richtung) quer zum Strahl geladener Teilchen verschiebbar.

Vorzugsweise sind das mindestens eine blockförmige Energiekorrekturelement und das mindestens eine keilförmige Energiekorrekturelement im Wesentlichen senkrecht zum Strahl geladener Teilchen verschiebbar.

Die Energiekorrektureinheit gemäß dieser Ausführungsform wird gemäß einem weiteren separaten Aspekt der Erfindung auch isoliert von den anderen Aspekten offenbart. Es wird insbesondere eine Energiekorrektureinheit offenbart, wobei die Energiekorrektureinheit mindestens ein quer zum Strahl geladener Teilchen verschiebbares blockförmiges Energiekorrekturelement und mindestens ein quer zum Strahl geladener Teilchen verschiebbares keilförmiges Energiekorrekturelement aufweist. Ebenfalls wird gemäß einem weiteren separaten Aspekt der Erfindung ein Strahlführungssystem offenbart, wobei das Strahlführungssystem eine Energiekorrektureinheit zur Einstellung der Energie der geladenen Teilchen des Strahls aufweist und die Energiekorrektureinheit mindestens ein quer zum Strahl geladener Teilchen verschiebbares blockförmiges Energiekorrekturelement und mindestens ein quer zum Strahl geladener Teilchen verschiebbares keilförmiges Energiekorrekturelement aufweist. Die Energiekorrektureinheit bzw. das Strahlführungssystem gemäß diesen separaten Aspekten können jedoch gemäß den vorherigen und folgenden vorteilhaften Ausführungsformen weiter ausgestaltet werden.

Gemäß einer bevorzugten Ausführungsform des erfindungsgemäßen Strahlführungssystems weist die Energiekorrektureinheit eine Mehrzahl von quer zum Strahl geladener Teilchen verschiebbaren blockförmigen Energiekorrekturelementen auf, welche unterschiedliche Einstellungen der Energie des Strahls geladener Teilchen ermöglichen.

Durch die Mehrzahl von (vorzugsweise unterschiedlichen) blockförmigen Energiekorrekturelementen kann eine flexible Einstellung der Energie der geladenen Teilchen des Strahls geladener Teilchen über einen weiten Energiebereich ermöglicht werden. Die blockförmigen Energiekorrekturelemente können beispielsweise in Richtung des Strahls geladener Teilchen gesehen unterschiedliche Ausdehnungen aufweisen und/oder aus unterschiedlichen Materialien bestehen.

Gemäß einer weiteren bevorzugten Ausgestaltung des erfindungsgemäßen Strahlführungssystems weist die Energiekorrektureinheit mehrere, insbesondere zwei quer zum Strahl geladener Teilchen verschiebbare keilförmige Energiekorrekturelemente auf.

Beispielsweise ist das Strahlführungssystem derart eingerichtet, dass die keilförmigen Energiekorrekturelemente gleichzeitig in den Strahl geladener Teilchen positioniert werden können. Beispielsweise ist ein keilförmiges Energiekorrekturelement aus einer ersten Richtung in den Strahl geladener Teilchen verschiebbar und ein weiteres keilförmiges Energiekorrekturelement aus einer zweiten Richtung (beispielsweise aus einer der ersten Richtung entgegengesetzten Richtung) in den Strahl geladener Teilchen verschiebbar.

Dadurch kann der Bereich, in dem eine Feinabstimmung möglich ist, erweitert werden. Zudem kann dadurch eine über den Querschnitt des Strahls geladener Teilchen asymmetrische Reduktion der Energie vermieden werden. Beispielsweise sind zwei keilförmige Energiekorrekturelemente hierzu spiegelsymmetrisch oder punktsymmetrisch zueinander angeordnet.

Gemäß einer weiteren bevorzugten Ausgestaltung des Strahlführungssystems ist die Energiekorrektureinheit zumindest teilweise aus Borcarbid hergestellt.

Im Gegensatz zu im Stand der Technik verwendeten Materialien kann durch die Verwendung von Borcarbid (B₄C) eine kompaktere und sicherere Energiekorrektureinheit bereitgestellt werden. Der hohe Anteil an Bor (Ordnungszahl 5) und die hohe Dichte von Borcarbid reduzieren die Aufweitung des Phasenraums im Vergleich zu bisher verwendeten Materialien. Beispielsweise kann im Vergleich zu aus (ausschließlich) Graphit bestehenden Energiekorrektureinheiten eine bis zu 30% kompaktere Energiekorrektureinheit bereitgestellt werden. Bevorzugt beträgt der Abstand vom Austrittsfenster der Strahlerzeugungseinheit (zum Beispiel ein Beschleuniger) bis zum Ende der Energiekorrektureinheit in Richtung des Strahls geladener Teilchen gesehen weniger als 2 m, insbesondere höchstens 1,7 m. Gleichzeitig kann durch die geringere Ausdehnung der Energiekorrektureinheit eine geringere Ausdehnung in Richtung des Strahls geladener Teilchen eine geringere Aufweitung des Strahls geladener Teilchen erreicht werden. Dies ermöglicht schließlich verbesserte Strahleigenschaften und verbesserte Transmissionseigenschaften des Strahlführungssystems. Zudem kann im Vergleich zu Beryllium enthaltenden Energiekorrektureinheit eine Energiekorrektureinheit mit geringerer Toxizität bereitgestellt werden.

Es ist ebenfalls möglich, eine Energiekorrektureinheit aus unterschiedlichen Materialien, beispielsweise Borcarbid und Graphit, vorzusehen. Beispielsweise können die blockförmigen und keilförmigen Energiekorrekturelemente aus unterschiedlichen Materialien bestehen.

Beispielsweise kann unter anderem durch den Einsatz einer derartigen Energiekorrektureinheit der Strahl geladener Teilchen mit einer Energie von 215MeV auf 90MeV abgebremst werden, wobei eine Transmission von fast 1% am Behandlungsort erreicht werden kann. Selbst bei einer Abbremsung auf 60MeV kann eine Transmission von noch etwa 0,3% erreicht werden. Im Vergleich zu Werten aus dem Stand der Technik sind dies deutlich höhere Werte.

Gemäß einer weiteren bevorzugten Ausgestaltung des erfindungsgemäßen Strahlführungssystems weist das Strahlführungssystem in Richtung des Strahls geladener Teilchen gesehen nach der Energiekorrektureinheit eine Kollimatoreinheit auf.

Hierdurch kann der Phasenraum nach der Energiekorrektureinheit eingegrenzt werden und die Strahlqualität am Behandlungsort verbessert werden. Vorzugsweise ist die Kollimatoreinheit unmittelbar nach der Energiekorrektureinheit angeordnet. Beispielsweise ist die Kollimatoreinheit als eine oder mehrere, bevorzugt zwei Blenden ausgebildet. Eine Blende ist beispielsweise ein Materialblock mit einer oder mehreren Öffnungen. Beispielsweise weisen die Blenden jeweils eine kreisförmige und/oder eine entgegen der Richtung des Strahls geladener Teilchen gesehen konisch zulaufende Öffnung auf. Der Strahl geladener Teilchen durchläuft die Kollimatoreinheit bevorzugt in einem Vakuum.

Die Kollimatoreinheit ist vorzugsweise im unbeweglichen Abschnitt (insbesondere nicht im beweglichen Traggestell) des Strahlführungssystems vorgesehen.

Gemäß einer weiteren bevorzugten Ausgestaltung des Strahlführungssystems weist das Strahlführungssystem zwischen der Energiekorrektureinheit und der in Richtung des Strahls geladener Teilchen gesehen ersten magnetischen Strahlablenkeinheit eine Driftstrecke auf, welche frei von magnetischen Strahlablenkeinheiten und/oder magnetischen Strahlformungseinheiten ist.

Durch die Driftstrecke kann vorteilhaft ein Bereich zur Verfügung gestellt werden, welcher beispielsweise eine oder mehrere Messeinrichtungen (insbesondere Strahlmonitore) zur Strahlkontrolle aufnehmen kann. Ebenfalls kann dieser Bereich zur Abschirmung (beispielsweise mittels einer Betonabschirmung) des Behandlungsortes von der Strahlerzeugungseinheit dienen. Beispielsweise beträgt die Länge der Driftstrecke mindestens 1 m und/oder höchstens 2 m. Die Driftstrecke ist bevorzugt zumindest teilweise im Vakuum vorgesehen, beispielsweise in einem Vakuumrohr von bevorzugt wenigen Zentimetern Durchmesser. Bevorzugt ist die Driftstrecke frei von jeglichen magnetischen Komponenten.

Die Driftstecke ist vorzugsweise im unbeweglichen Abschnitt (insbesondere nicht im beweglichen Traggestell) des Strahlführungssystems vorgesehen.

Die Driftstrecke gemäß dieser Ausführungsform wird gemäß einem weiteren separaten Aspekt der Erfindung auch isoliert von den anderen Aspekten offenbart. Es wird insbesondere ein Strahlführungssystem mit einer Energiekorrektureinheit und mindestens einer Strahlablenkeinheit offenbart, wobei das Strahlführungssystem zwischen der Energiekorrektureinheit und der in Richtung des Strahls geladener Teilchen gesehen ersten magnetischen Strahlablenkeinheit eine Driftstrecke aufweist, welche frei von magnetischen Strahlablenkeinheiten und/oder magnetischen Strahlformungseinheiten ist. Das Strahlführungssystem gemäß diesem separaten Aspekt kann jedoch gemäß den vorherigen und folgenden vorteilhaften Ausführungsformen weiter ausgestaltet werden.

Gemäß einem weiteren Aspekt der vorliegenden Erfindung wird die eingangs genannte Aufgabe durch eine Teilchenstrahl-Therapieanlage gelöst mit einer Strahlerzeugungseinheit zur Erzeugung eines Strahls geladener Teilchen, insbesondere Ionen, vorzugsweise Protonen, und mit einem Strahlführungssystem gemäß den unterschiedlichen Aspekten, wobei vorzugsweise in Richtung des Strahls der geladenen Teilchen gesehen hinter der Strahlerzeugungseinheit eine Strahlformungseinheit vorgesehen wird.

Wie bereits bezüglich des erfindungsgemäßen Strahlführungssystems ausgeführt, können durch das Strahlführungssystem Teilchenstrahl-Therapieanlagen mit verbesserten Strahleigenschaften und gleichzeitig kompakteren Bauweisen bereitgestellt werden, welche im Stand der Technik bisher nicht erreichbar waren.

Beispielsweise wird eine Strahlerzeugungseinheit mit einem Durchmesser von weniger als 4 m verwendet. Die Strecke von der Strahlerzeugungseinheit bis zum Ende der Energiekorrektureinheit kann vorteilhaft weniger als 2 m betragen. Die Strecke vom Ende der Energiekorrektureinheit bis zum Behandlungsort kann bevorzugt weniger als 9 m betragen. Durch die unterschiedlichen Aspekte ist es daher möglich, dass die Länge der Teilchenstrahl-Therapieanlage bis zum Behandlungsort gemessen weniger als 14 m beträgt.

Beispielsweise ist die Strahlerzeugungseinheit eine Beschleunigereinrichtung, beispielsweise eine Beschleunigereinrichtung, welche einen Strahl geladener Teilchen mit konstanter Energie erzeugt, beispielsweise ein Zyklotron. Es ist jedoch auch denkbar, dass die Strahlerzeugungseinrichtung eine Beschleunigereinrichtung ist, welche einen Strahl geladener Teilchen mit variabler Energie erzeugt, beispielsweise ein Synchrotron. Wie bereits zuvor ausgeführt, wird bevorzugt eine Strahlerzeugungseinheit verwendet, welche geladene Teilchen mit einer kinetischen Energie von mehr als 200 MeV emittiert, beispielsweise zwischen 200 und 300 MeV, insbesondere zwischen 210 und 250 MeV.

Ist in Richtung des Strahls geladener Teilchen gesehen hinter der Strahlerzeugungseinheit wenigstens eine Strahlformungseinheit vorgesehen, können die Strahleigenschaften und/oder die Transmission verbessert werden. Dies ist darauf zurückzuführen, dass durch wenigstens eine Strahlformungseinheit eine Abbildung des von der Strahlerzeugungseinheit emittierten Phasenraums auf die vorzugsweise (unmittelbar) dahinter angeordnete Energiekorrektureinheit derart erfolgen kann, dass ein möglichst großer (im Optimalfall der gesamte) Teil des Phasenraums geladene Teilchen enthält, welche zur Transmission bis zum Behandlungsort beitragen. Wie bereits zuvor ausgeführt, können die Strahlformungseinheiten insbesondere magnetische Quadrupole (beispielsweise drei) sein.

Ebenfalls kann ein Solenoid-Magnet als Strahlformungseinheit hinter der Strahlerzeugungseinheit vorgesehen sein.

Die Teilchenstrahl-Therapieanlage dieser Ausführungsform wird gemäß einem weiteren separaten Aspekt der Erfindung auch isoliert von den anderen Aspekten offenbart. Es wird insbesondere eine Teilchenstrahl-Therapieanlage offenbart mit einer Strahlerzeugungseinheit zur Erzeugung des Strahls geladener Teilchen, insbesondere Ionen, vorzugsweise Protonen, und mit einem Strahlführungssystem, wobei in Richtung des Strahls geladener Teilchen gesehen hinter der Strahlerzeugungseinheit wenigstens eine Strahlformungseinheit vorgesehen ist. Die Teilchenstrahl-Therapieanlage gemäß diesem separaten Aspekt kann jedoch gemäß den vorherigen und folgenden vorteilhaften Ausführungsformen weiter ausgestaltet werden.

Gemäß einem weiteren Aspekt der Erfindung wird die eingangs genannte Aufgabe auch durch ein Verfahren gelöst umfassend die Schritte: Erzeugen eines Strahls geladener Teilchen, insbesondere Ionen, vorzugsweise Protonen, und Führen des Strahls geladener Teilchen mittels eines Strahlführungssystems gemäß der unterschiedlichen beschriebenen Aspekte.

Bezüglich der Vorteile und weiteren Ausgestaltungen des Verfahrens wird auf die vorherigen Aspekte und deren Ausgestaltungen verwiesen.

Insbesondere soll durch die vorherige und folgende Beschreibung von Mitteln zur Durchführung eines Verfahrensschrittes der entsprechende Verfahrensschritt offenbart sein. Ebenfalls sollen durch die Offenbarung von Verfahrensschritten auch entsprechende Mittel oder Einrichtungen zur Durchführung der Verfahrensschritte offenbart sein.

Die oben beschriebenen Ausführungsbeispiele und beispielhaften Ausgestaltungen aller Aspekte der vorliegenden Erfindung sollen auch in allen Kombinationen miteinander offenbart verstanden werden.

Weitere vorteilhafte beispielhafte Ausgestaltungen der unterschiedlichen Aspekte sind der folgenden detaillierten Beschreibung einiger beispielhafter Ausführungsformen der Aspekte, insbesondere in Verbindung mit den Figuren zu entnehmen. Die der Anmeldung beiliegenden Figuren sollen jedoch nur dem Zwecke der Verdeutlichung, nicht aber zur Bestimmung des Schutzbereiches der Erfindung dienen. Die beiliegenden Zeichnungen sind nicht notwendigerweise maßstabsgetreu und sollen lediglich das allgemeine Konzept der vorliegenden Aspekte beispielhaft widerspiegeln. Insbesondere sollen Merkmale, die in den Figuren enthalten sind, keineswegs als notwendiger Bestandteil der vorliegenden Erfindung erachtet werden.

Es zeigen:
- Fig. 1: eine schematische Schnittansicht eines Ausführungsbeispiels einer Teilchenstrahl-Therapieanlage mit einem Ausführungsbeispiel eines Strahlführungssystems gemäß der vorliegenden Erfindung;
- Fig. 2: eine schematische Schnittansicht eines weiteren Ausführungsbeispiels des beweglichen Abschnitts eines Strahlführungssystems gemäß der vorliegenden Erfindung;
- Fig. 3: eine schematische Schnittansicht eines Ausführungsbeispiels einer Energiekorrektureinheit.

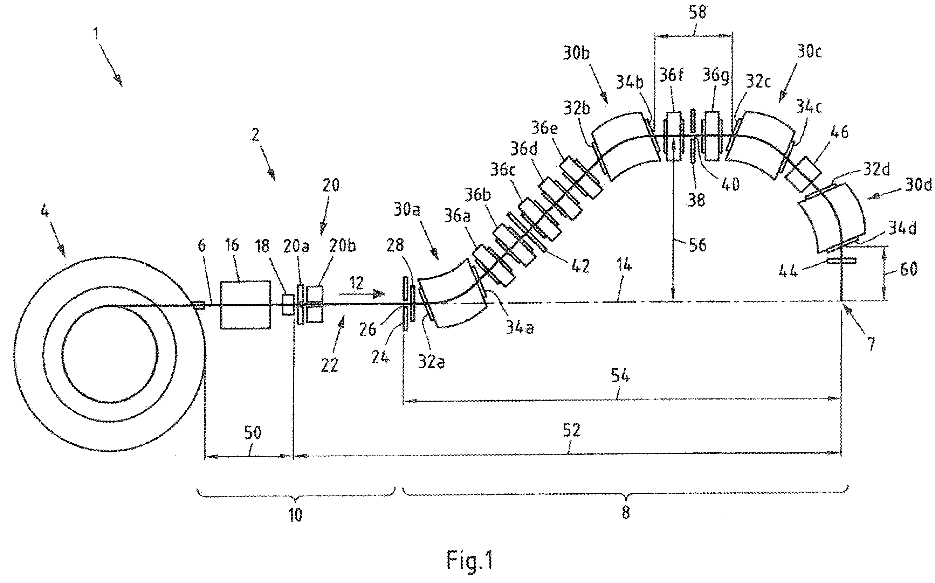

Fig. 1 zeigt eine schematische Schnittansicht eines Ausführungsbeispiels einer Teilchenstrahl-Therapieanlage 1 mit einem Ausführungsbeispiel eines Strahlführungssystems 2. Die Teilchenstrahl-Therapieanlage umfasst eine Strahlerzeugungseinheit 4 zur Erzeugung eines Strahls geladener Teilchen 6, welche insbesondere Protonen sein können. Der Strahl geladener Teilchen 6 wird durch das Strahlführungssystem 2 an einen Behandlungsort 7 geführt. Das Strahlführungssystem 2 weist einen beweglichen, in diesem Fall einen um 360° rotierbaren Abschnitt 8 und einen unbeweglichen Abschnitt 10 auf. Der rotierbare Abschnitt 8 kann beispielsweise durch ein Traggestell in Form einer Gantry (nicht dargestellt) realisiert sein.

Die Strahlerzeugungseinheit 4 ist in diesem Fall ein Zyklotron, das heißt eine Beschleunigereinrichtung, welche den Strahl geladener Teilchen 6 mit konstanter Energie erzeugt. Die Strahlerzeugungseinheit 4 emittiert geladene Teilchen mit einer konstanten kinetischen Energie, beispielsweise 210 MeV, 215 MeV oder 250 MeV. Es hat sich gezeigt, dass mittels geladener Teilchen mit einer kinetischen Energie von etwa 207MeV etwa 95%, mittels geladener Teilchen mit einer kinetischen Energie von etwa 198 MeV noch etwa 90% der zu behandelnden Patienten behandelt werden können.

Der Strahl geladener Teilchen verläuft zunächst in Richtung des Pfeils 12 entlang einer ursprünglichen Achse 14 des Strahls geladener Teilchen 6.

In Richtung 12 des Strahls geladener Teilchen 6 gesehen hinter der Strahlerzeugungseinheit 4 ist beispielsweise eine Strahlformungseinheit oder Strahlformungseinheiten 16 vorgesehen. Die Strahlformungseinheit 16 kann beispielsweise durch einen oder mehrere Solenoid-Magneten oder durch mehrere (beispielsweise drei) Quadrupolmagnete realisiert sein. Die Fokussierung durch die Strahlformungseinheit 16dient dazu, eine optimale Abbildung des von der Strahlerzeugungseinheit emittierten Phasenraums auf die dahinter angeordnete Energiekorrektureinheit derart zu ermöglichen, dass ein möglichst großer (im Optimalfall der gesamte) Teil des Phasenraums geladene Teilchen enthält, welche zur Transmission bis zum Behandlungsort beitragen. Dadurch kann die Strahlqualität und Transmission des Strahls geladener Teilchen 6 bis zu dem Behandlungsort 7 erhöht werden.

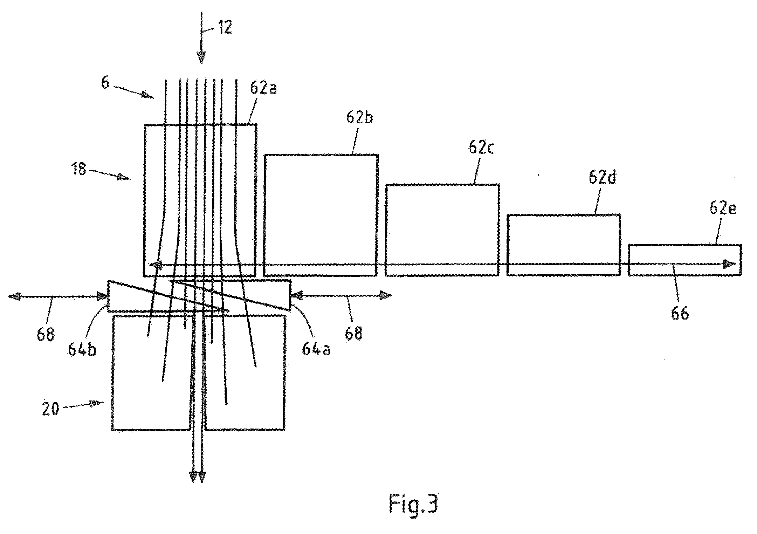

Der Strahl geladener Teilchen 6 wird anschließen durch eine Energiekorrektureinheit 18 geführt. Durch die Energiekorrektureinheit 18 kann eine Einstellung der Energie der geladenen Teilchen des Strahls geladener Teilchen 6 erfolgen. Die Energiekorrektureinheit wird in Zusammenhang mit Fig. 3 näher beschrieben.

Das Strahlführungssystem weist zudem in Richtung 12 des Strahls geladener Teilchen 6 gesehen nach der Energiekorrektureinheit 18 eine Kollimatoreinheit 20 auf, welche der Strahl der geladenen Teilchen 6 anschließend durchläuft. Die Kollimatoreinheit umfasst zwei Blenden 20a, 20b welche jeweils als Materialblock mit jeweils einer kreisförmigen und entgegen der Richtung 12 des Strahls geladener Teilchen 6 gesehen konisch zulaufenden Öffnung ausgebildet sind.

Das Strahlführungssystem 2 weist nach der Energiekorrektureinheit 18 und der Kollimatoreinheit 20 in Richtung 12 des Strahls geladener Teilchen 6 gesehen eine Driftstrecke 22 auf. Die Driftstrecke 22 ist frei von magnetischen Einheiten, wie magnetische Strahlablenkeinheiten oder magnetischen Strahlformungseinheiten. Im Bereich der Driftstrecke 22 kann eine Abschirmung, etwa eine Betonabschirmung (nicht dargestellt) vorgesehen sein. Zudem können in dem Bereich der Driftstrecke 22 Messeinrichtungen wie Strahlmonitore (nicht dargestellt) vorgesehen sein.

Der Strahl geladener Teilchen 6 durchläuft den Abschnitt der Energiekorrektureinheit 18, der Kollimatoreinheit 20 und der Driftstrecke 22 des Strahlführungssystems 2 in einem Vakuum, was die Strahleigenschaften und die Transmission in diesem Abschnitt verbessert.

Die bisher beschriebenen Elemente des Strahlführungssystems 2 sind in dem unbeweglichen Abschnitt 10 des Strahlführungssystems 2 angeordnet. Um den Behandlungsort 7 aus möglichst vielen Winkeln bestrahlen zu können, ist der rotierbare Abschnitt 8 vorgesehen. Die Rotationsachse des Traggestells (nicht dargestellt) fällt mit der ursprünglichen Achse 14 des Strahls geladener Teilchen6 zusammen.

In dem rotierbaren Abschnitt 8 weist das Strahlführungssystem zunächst eine Kollimatoreinheit 24 auf. Durch die Kollimatoreinheit 24 kann der Phasenraum des Strahls geladener Teilchen definiert werden. Die Kollimatoreinheit 24 ist hier als Blende ausgebildet, in diesem Fall als Materialblock mit einer rechteckigen Öffnung 26. Die Geometrie der Öffnung 26 lässt sich in beide Richtungen transversal zum Strahl geladener Teilchen 6 verändern, sodass eine flexible Anpassung der Strahleigenschaften erfolgen kann.

Anschließend ist ein optionaler Strahlmonitor 28 vorgesehen, um die Strahleigenschaften zu überprüfen. Der Strahlmonitor kann jedoch auch an anderen Stellen des Strahlführungssystems 2 vorgesehen werden.

Anschließend weist das Strahlführungssystem 2 zur Führung des Strahls geladener Teilchen 6 mehrere, in diesem Fall vier als magnetische Dipole ausgebildete magnetische Strahlablenkeinheiten 30a, 30b, 30c, 30d auf. Die magnetischen Strahlablenkeinheiten 30a, 30b, 30c, 30d weisen jeweils eine Eintrittsseite 32a, 32b, 32c, 32d zum Eintreten des Strahls geladener Teilchen 6 unter einer Eintrittsrichtung in die jeweilige magnetische Strahlablenkeinheit auf. Die magnetische Strahlablenkeinheiten 30a, 30b, 30c, 30d weisen zudem jeweils eine Austrittsseite 34a, 34b, 34c, 34d zum Austreten des Strahls geladener Teilchen 6 unter einer Austrittrichtung aus der magnetische Strahlablenkeinheit 30a, 30b, 30c, 30d auf. Die Eintrittsseiten 32a, 32b, 32c, 32d sind jeweils parallel zu der jeweiligen Austrittsseite 34a, 34b, 34c, 34d ausgebildet. Dabei sind die magnetischen Strahlablenkeinheit 30a, 30b, 30c, 30d jeweils derart zur Ablenkung des Strahls geladener Teilchen 6 in dem Strahlführungssystem 2 vorgesehen, dass die jeweiligen Eintrittsseite 32a, 32b, 32c, 32d schräg zur jeweiligen Eintrittsrichtung des Strahls geladener Teilchen 6 und die jeweiligen Austrittsseite 34a, 34b, 34c, 34d schräg zur jeweiligen Austrittsrichtung des Strahls geladener Teilchen 6 liegt. Dabei sind die magnetischen Strahlablenkeinheiten 30a, 30b, 30c, 30d in diesem Fall derart zur Ablenkung des Strahls geladener Teilchen 6 in dem Strahlführungssystem 2 vorgesehen, dass die Eintrittsrichtung und die Austrittsrichtung bei jeder Strahlablenkeinheit 30a, 30b, 30c, 30d in einem Winkel von 45° zueinander stehen. Die Strahlablenkeinheiten 30a, 30b, 30c, 30d sind dabei symmetrisch in dem Strahl geladener Teilchen positioniert, das heißt der Winkel zwischen Eintrittsrichtung und Eintrittsseite 32a, 32b, 32c, 32d und der Winkel zwischen Austrittsrichtung und Austrittsseite 34a, 34b, 34c, 34d sind bei den einzelnen Strahlablenkeinheiten 30a, 30b, 30c, 30d jeweils identisch.

Der Strahl geladener Teilchen 6 wird durch die erste Strahlablenkeinheit 30a von der ursprünglichen Achse 14 des Strahls geladener Teilchen weggelenkt. Der Strahl geladener Teilchen 6 wird durch die zweite Strahlablenkeinheit 30b wieder zu der ursprünglichen Achse 14 hingelenkt und verläuft dann parallel zu der ursprünglichen Achse 14 des Strahls geladener Teilchen 6. Anschließend wird der Strahl geladener Teilchen 6 durch die dritte und die vierte Strahlablenkeinheit 30c, 30d ebenfalls wieder zu der ursprünglichen Achse 14 hingelenkt, sodass der Strahl geladener Teilchen 6 nach der letzten Strahlablenkeinheit 30d senkrecht zu der ursprünglichen Achse 14 des Strahls geladener Teilchen 6 verläuft und die ursprüngliche Achse 14 kreuzt.

Durch die beschriebene Ausbildung Strahlablenkeinheiten 30a, 30b, 30c, 30d können verbesserte Strahleigenschaften bei einem gleichzeitig kompakteren Strahlführungssystem 2 erreicht werden. Dies wird unter anderem darauf zurückgeführt, dass die magnetischen Strahlablenkeinheiten 30a, 30b, 30c, 30d nicht nur ein Ablenken des Strahls geladener Teilchen sondern zudem auch eine Fokussierung des Strahls geladener Teilchen ähnliche einem Quadrupolmagneten erreichen können.

Die vorgesehenen magnetischen Strahlablenkeinheiten 30a, 30b, 30c, 30d weisen dabei eine Defokussierung in einer transversalen Richtung (hier in der Zeichenebene) und eine Fokussierung in einer dazu senkrechten Richtung auf. In diesem Punkt haben die magnetischen Strahlablenkeinheiten 30a, 30b, 30c, 30d ähnliche Eigenschaften wie Quadrupolmagnete, die ebenfalls in einer transversalen Richtung fokussieren und in der dazu senkrechten Richtung defokussieren. Alle vier magnetischen Strahlablenkeinheiten 30a, 30b, 30c, 30d fokussieren also nur in einer Richtung (senkrecht zur Zeichenebene). Von den vorgesehenen sieben Quadrupolmagneten fokussieren daher fünf in der transversalen Richtung in der Zeichenebene und nur zwei in der dazu senkrechten Richtung. Insgesamt wird so eine ausreichende Fokussierung in beiden transversalen Koordinatenrichtungen erreicht. Im Endeffekt sind durch die Fokussierung der Dipole in y-Richtung also nur noch 2 Quadrupole mit Fokussierung in der gleichen Richtung erforderlich.

Zudem kann das Herstellungsverfahren der Strahlablenkeinheiten 30a, 30b, 30c, 30d aufgrund der parallelen Eintritts- und Austrittsseiten vereinfacht werden, da der Eisenkern der Strahlablenkeinheiten 30a, 30b, 30c, 30d durch parallel aufeinander geschichtete Platten hergestellt werden kann.

Das Strahlführungssystem 2 weist zudem mehrere, in diesem Fall sieben, magnetische Strahlformungseinheiten 36a, 36b, 36c, 36d, 36e, 36f, 36g in Form von Quadrupolmagneten auf. Durch die Strahlformungseinheiten 36a, 36b, 36c, 36d, 36e, 36f, 36g kann die Strahleigenschaft des Strahls geladener Teilchen 6 weiter verbessert werden. Insbesondere ist unter anderem aufgrund der vorteilhaften Strahlablenkeinheiten 30a, 30b, 30c, 30d nur eine vergleichsweise geringe Anzahl von Strahlformungseinheiten 36a, 36b, 36c, 36d, 36e, 36f, 36g notwendig, um gute Strahleigenschaften zu erzielen, was ein kompaktes Strahlführungssystem 2 erlaubt.

Zwischen der (in Richtung 12 des Strahls geladener Teilchen 6 gesehen) ersten magnetischen Strahlablenkeinheit 30a und der zweiten magnetischen Strahlablenkeinheit 30b sind die fünf Strahlformungseinheiten 36a, 36b, 36c, 36d, 36e vorgesehen. Zwischen der zweiten magnetischen Strahlablenkeinheit 30b und der dritten magnetischen Strahlablenkeinheit 30c sind weitere zwei Strahlformungseinheiten 36f, 36g vorgesehen. Die Strahlformungseinheiten 36a, 36b, 36c, 36d, 36e, 36f, 36g sind in diesem Fall alle gleich dimensioniert.

Das Strahlführungssystem 2 weist zwischen den zwei magnetischen Strahlablenkeinheiten 30b, 30c eine weitere Kollimatoreinheit 38 in Form einer Blende mit einer rechteckige Öffnung 40 auf. Die Öffnung lässt sich in beide Richtungen transversal zum Strahl geladener Teilchen 6 verändern, wodurch eine Formgebung für den Strahlfleck am Behandlungsort 7 erfolgen kann. Es hat sich gezeigt, dass zwischen den Strahlablenkeinheiten 30b, 30c eine vergleichsweise große Impulsdispersion herrscht. Dieser kann durch das Vorsehen der Kollimatoreinheit 38 entgegengewirkt werden. Durch die Kollimatoreinheit 38 kann nämlich eine Impulsselektion für den Strahl geladener Teilchen 6 am Behandlungsort 7 erreichen werden.

In dem Strahlführungssystem 2 sind weiterhin die Strahlmonitore 42 und 44 vorgesehen. Der Strahlmonitor 42 ist zwischen den Strahlformungseinheiten 36b und 36c vorgesehen. Der Strahlmonitor 44 ist nach der vierten Strahlablenkeinheit 30d und vor dem Behandlungszentrum 7 vorgesehen.

Weiterhin weist das Strahlführungssystem zwischen der Strahlablenkeinheit 30c und der Strahlablenkeinheit 30d einen Scanmagneten 46 auf. Der Scanmagnet kann vorteilhaft an dieser Position eingesetzt werden, da hierdurch am Behandlungsort ein großer Scanbereich abgedeckt werden kann. Mit einem System wie beispielsweise bereits 2005 von V. Anferov vorgestellt, lässt sich zum Beispiel der Strahl derart verschieben, dass am Behandlungsort ein Bereich von 210 mm mal 175 mm überdeckt werden kann, bei einem Ablenkwinkel von nur ± 44 mrad in beiden Koordinatenrichtungen. Eine weitere Vergrößerung des Scanbereichs ist möglich. Da die Strahlablenkeinheit 30d den durch den Scanmagneten 46 abgelenkten geladener Teilchen 6 aufnehmen muss, kann die Strahlablenkeinheit 30d eine im Vergleich zu den übrigen Strahlablenkeinheiten 30a, 30b, 30c vergrößerte Eintrittsöffnung und/oder Austrittsöffnung aufweisen. Die Strahlablenkeinheiten 30a, 30b, 30c können baugleich sein.

Dadurch, dass der Scanmagnet 46 zwischen der letzten und vorletzten (das heißt der dritten und der vierten) Strahlablenkeinheit 30c, 30d vorgesehen ist, kann ein großer Scan-Bereich am Behandlungsort 7 ermöglicht werden.

Es hat sich gezeigt, dass das Strahlführungssystem 2 besonders kompakt vorgesehen werden kann. Die Strecke 50 von der Strahlerzeugungseinheit 4 bis zum Ende der Energiekorrektureinheit 18 beträgt hier weniger als 2 m. Die Strecke 52 vom Ende der Energiekorrektureinheit 18 bis zum Behandlungsort 7 beträgt hier weniger als 9 m. Der Strahl geladener Teilchen 6 kann hier mit dem Strahlführungssystem 2 auf einer Strecke 54 von weniger als 8 m von der Richtung 12 entlang der ursprünglichen Achse 14 des Strahl geladener Teilchen 6 zum Behandlungsort 7 geführt werden. Dabei beträgt der maximale Abstand 56 des Strahls geladener Teilchen 6 weniger als 3 m von der ursprünglichen Achse 14 des Strahls geladener Teilchen 6. Der Abstand 58 der zweiten und dritten magnetischen Strahlablenkeinheit 30b, 30c beträgt dabei weniger als 1,5 m. Der Abstand 60 der letzten magnetische Strahlablenkeinheit 30d vom Behandlungsort beträgt dabei weniger als 1 m, beispielsweise 0,991m.

Zu beachten ist, dass sich die geometrischen Abmessungen auf einen Strahl geladener Teilchen mit einer kinetischen Energie von etwa 210MeV beziehen. Werden höhere Energien verwendet, werden die geometrischen Abmessungen bevorzugt mit einem Faktor multipliziert. Dieser geometrische Skalenfaktor ist beispielsweise gerade das Verhältnis der Impulse von Protonen höherer Energie (zum Beispiel 245 MeV) zu 210 MeV Protonen.

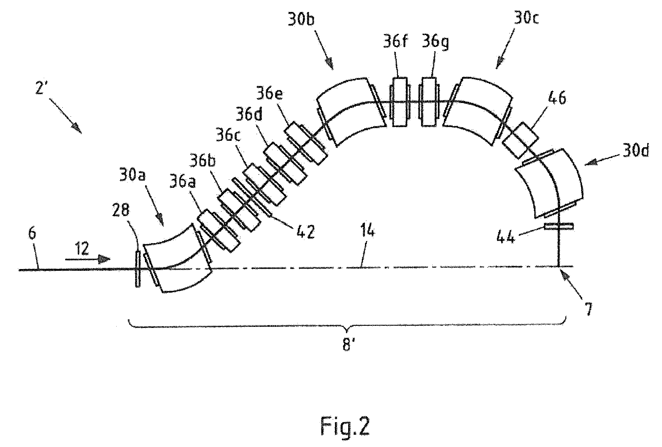

Fig. 2 zeigt eine schematische Schnittansicht eines weiteren Ausführungsbeispiels eines Strahlführungssystems 2'. Der gezeigte rotierbare Abschnitt 8' des Strahlführungssystems 2' aus Fig. 2 ist ähnlich zu dem rotierbaren Abschnitt 8 des Strahlführungssystems 2 aus Fig. 1. Insofern werden für gleiche Elemente gleiche Bezugszeichen verwendet. Ebenfalls wird auf die Ausführungen zu dem Strahlführungssystem 2 aus Fig.1 verwiesen. Insbesondere kann der rotierbare Abschnitt 8' anstelle des rotierbaren Abschnitts 8 in dem Strahlführungssystem 2 vorgesehen werden. Im Folgenden soll lediglich auf die Unterschiede zu dem Strahlführungssystem 2 aus Fig. 1 eingegangen werden.

Der wesentliche Unterschied zwischen dem Strahlführungssystem 2 und dem Strahlführungssystem 2' ist, dass das Strahlführungssystem 2' keine Kollimatoreinheiten 24, 38 in dem rotierbaren Abschnitt 8' aufweist. Dadurch kann insbesondere der Abstand der magnetischen Strahlablenkeinheiten 30b, 30c verkürzt werden, sodass deren Abstand beispielsweise nur noch weniger als 1,2 m, insbesondere weniger als 1,1 m betragen kann. Die Selektion des Phasenraums erfolgt in diesem Fall bereits vor Eintritt des Strahls geladener Teilchen 6 in den rotierbaren Abschnitt 8'.

Fig. 3 zeigt eine schematische Schnittansicht eines Ausführungsbeispiels einer Energiekorrektureinheit 18 in Form eines Degraders, wie er beispielsweise in dem Strahlführungssystem 2 oder 2' verwendet werden kann. Die Energiekorrektureinheit 18 weist eine Mehrzahl von quer zum Strahl geladener Teilchen 6 verschiebbaren blockförmigen Energiekorrekturelementen 62a, 62b, 62c, 62d, 62e und zwei quer zum Strahl geladener Teilchen 6 verschiebbare keilförmige Energiekorrekturelemente 64a, 64b auf.

Die blockförmigen Energiekorrekturelemente 62a, 62b, 62c, 62d, 62e sind hier entlang des Pfeils 66 senkrecht zum Strahl geladener Teilchen 6 verschiebbar. Dadurch können unterschiedliche Einstellungen der Energie der geladenen Teilchen des Strahls geladener Teilchen 6 ermöglicht werden, je nachdem welches der blockförmigen Energiekorrekturelemente 62a, 62b, 62c, 62d, 62e in den Strahl geladener Teilchen geschoben wird. Die blockförmigen Energiekorrekturelemente 62a, 62b, 62c, 62d, 62e weisen hierzu in Richtung 12 des Strahls geladener Teilchen 6 gesehen unterschiedliche Ausdehnungen auf. Die blockförmigen Energiekorrekturelemente 62a, 62b, 62c, 62d, 62e dienen hier einer groben Einstellung der Energie der geladenen Teilchen des Strahls geladener Teilchen 6, indem die Energie der geladenen Teilchen des Strahls geladener Teilchen 6 durch die blockförmigen Energiekorrekturelemente 62a, 62b, 62c, 62d, 62e auf diskrete Werte eingestellt werden kann.

Die keilförmigen Energiekorrekturelemente 64a, 64b sind entlang der Pfeile 68 ebenfalls senkrecht zum Strahl geladener Teilchen 6 verschiebbar. Die keilförmigen Energiekorrekturelement 64a, 64b dienen einer Feineinstellung der Energie der geladenen Teilchen des Strahls geladener Teilchen 6 nachdem der Strahl geladener Teilchen 6 durch eines der blockförmigen Energiekorrekturelemente 62a, 62b, 62c, 62d, 62e geführt wurde. Die Energie der geladenen Teilchen des Strahls geladener Teilchen 6 kann durch die keilförmigen Energiekorrekturelement 64a, 64b in einem begrenzten Bereich kontinuierlich eingestellt werden. Durch die Verschiebung der keilförmigen Energiekorrekturelemente quer zum Strahl geladener Teilchen wird die in Richtung des Strahls geladener Teilchen 6 gesehene Ausdehnung des keilförmigen Energiekorrekturelements 64a, 64b im Bereich des Strahls geladener Teilchen verändert. Die zwei keilförmigen Energiekorrekturelemente 64a, 64b sind vorliegend punktsymmetrisch zueinander angeordnet. Die angeschrägten Flächen der keilförmigen Energiekorrekturelemente 64a, 64b sind einander zugewandt. Durch diese Anordnung kann eine über den Querschnitt des Strahls geladener Teilchen 6 asymmetrische Reduktion der Energie vermieden werden.

Die blockförmigen Energiekorrekturelemente 62a, 62b, 62c, 62d, 62e der Energiekorrektureinheit 18 sind aus Graphit und/oder aus Borcarbid hergestellt. Ebenfalls ist jedoch denkbar, blockförmige Energiekorrekturelemente unterschiedlicher Materialien vorzusehen. Auch die keilförmigen Energiekorrekturelemente 64a, 64b der Energiekorrektureinheit 18 sind aus Graphit und/oder aus Borcarbid hergestellt. Auch hier ist denkbar, keilförmige Energiekorrekturelemente unterschiedlicher Materialien vorzusehen.

Aufgrund der Kompaktheit der Energiekorrektureinheit 18 kann ein übermäßiges Aufweiten des Phasenraums des Strahls geladener Teilchen 6 vermieden werden.

In Richtung 12 des Strahls geladener Teilchen 6 gesehen hinter der Energiekorrektureinheit 18 ist ein Kollimator 20 vorgesehen. Dieser kann beispielsweise der in Fig. 1 gezeigte Kollimator 20 oder ein Teil hiervon, etwa Kollimatoreinheit 20a sein.

Zusammenfassend ist es durch die unterschiedlichen Aspekte möglich, mittels kleiner Ablenkwinkel, verursacht durch das Magnetfeld des Scanmagneten 46, am Behandlungsort 7 einen großen Scanbereich zu erreichen und gleichzeitig die Abstände aller magnetischen Elemente von der Rotationsachse 14 gering zu halten.

Die Leistungsfähigkeit der Teilchenstrahl-Therapieanlage aus Fig. 1 kann mittels genauer numerischer Monte-Carlo-Berechnungen ermittelt werden. Beispielsweise beträgt die Transmissionseffizienz am Behandlungsort 7 bei Abbremsung einer von einem Zyklotron emittierten Energie von 215 MeV auf 90 MeV noch etwa 1%, bei einer Abbremsung auf 60 MeV noch 0,3%. Die Breite des Strahlflecks kann dabei auf 4 mm (1σ) eingestellt werden bei sehr geringer Elliptizität. Die Impulsbreite der am Behandlungsort 7 eintreffenden geladenen Teilchen lässt sich zwischen 2 und 7 Promille variieren. Dabei bleibt die Größe des Strahlflecks am Behandlungsort 7 erhalten. Selbst bei einer von der Strahlerzeugungseinheit emittierten Energie von 250 MeV und Abbremsung durch die Energiekorrektureinheit 18 auf 90 MeV beträgt die Transmissionseffizienz noch bis zu 0,4%.

Die physikalischen Grundlagen der Wechselwirkung von Protonen mit Materie und die zur Berechnung der Eigenschaften von Strahlführungssystemen verwendeten Programme sind in den folgenden wissenschaftlichen Berichten beschrieben:
- Particle Data Group, W.-M. Yao et al., "The Review of Particle Physics", Journal of Physics G33 (2006) 1 and update 2008.
- Karl L. Brown, Sam K. Howry, "TRANSPORT, A Computer Program for Designing Charged Particle Beam Transport Systems", SLAC Report No. 91 (1970) and later updates of the TRANSPORT program by U. Rohrer and others.
- U. Rohrer, "PSI Graphic TURTLE Framework based on a CERN-SLAC-FERMILAB version by K.L. Brown et al.", http://aea.web.psi.ch/Urs_Rohrer/MyWeb/turtle.htm.
- J. Drees, "Passage of Protons through Thick Degraders", Cryoelectra Report Sept. 2008.

Das Prinzip eines x-y Scan-Magneten ist in folgendem wissenschaftlichen Bericht beschrieben:
- V. Anferov, "Combined X-Y scanning magnet for conformal proton radiation therapy", Med. Phys. 32 (3), March 2005.

## Patentansprüche

1. Strahlführungssystem (2, 2') zur Führung eines Strahls geladener Teilchen (6) mit einer magnetischen Strahlablenkeinheit (30a, 30b, 30c, 30d),
wobei die magnetische Strahlablenkeinheit (30a, 30b, 30c, 30d) eine Eintrittsseite (32a, 32b, 32c, 32d) zum Eintreten des Strahls geladener Teilchen unter einer Eintrittsrichtung in die magnetische Strahlablenkeinheit (30a, 30b, 30c, 30d) aufweist,
wobei die magnetische Strahlablenkeinheit (30a, 30b, 30c, 30d) eine Austrittsseite (34a, 34b, 34c, 34d) zum Austreten des Strahls geladener Teilchen unter einer Austrittrichtung aus der magnetische Strahlablenkeinheit (30a, 30b, 30c, 30d) aufweist, und
wobei die Eintrittsseite (32a, 32b, 32c, 32d) der magnetischen Strahlablenkeinheit (30a, 30b, 30c, 30d) zumindest abschnittsweise im Wesentlichen parallel zu der Austrittsseite (34a, 34b, 34c, 34d) der magnetischen Strahlablenkeinheit (30a, 30b, 30c, 30d) ausgebildet ist.

2. Stahlführungssystem nach Anspruch 1,
wobei die magnetische Strahlablenkeinheit (30a, 30b, 30c, 30d) derart zur Ablenkung des Strahls geladener Teilchen (6) in dem Strahlführungssystem (2, 2') vorgesehen ist, dass die Eintrittsseite (32a, 32b, 32c, 32d) schräg zur Eintrittsrichtung des Strahls geladener Teilchen und/oder die Austrittsseite (34a, 34b, 34c, 34d) schräg zur Austrittsrichtung des Strahls geladener Teilchen (6) liegt.

3. Strahlführungssystem nach Anspruch 1 oder 2,
wobei die magnetische Strahlablenkeinheit (30a, 30b, 30c, 30d) derart zur Ablenkung des Strahls geladener Teilchen (6) in dem Strahlführungssystem (2, 2') vorgesehen ist, dass die Eintrittsrichtung und die Austrittsrichtung in einem Winkel von 30° bis 60°, vorzugsweise von 40° bis 50°, insbesondere im Wesentlichen 45°, zueinander stehen.

4. Strahlführungssystem nach einem der Ansprüche 1 bis 3,
wobei die magnetische Strahlablenkeinheit (30a, 30b, 30c, 30d) ein Dipolmagnet ist.

5. Strahlführungssystem nach einem der Ansprüche 1 bis 4,
wobei das Strahlführungssystem (2, 2') mehrere, insbesondere vier magnetische Strahlablenkeinheiten (30a, 30b, 30c, 30d) aufweist.

6. Strahlführungssystem nach einem der Ansprüche 1 bis 5,
wobei das Strahlführungssystem (2, 2') wenigstens eine magnetische Strahlformungseinheit (36a, 36b, 36c, 36d, 36e, 36f, 36g), insbesondere mehrere magnetische Strahlformungseinheiten (36a, 36b, 36c, 36d, 36e, 36f, 36g), vorzugsweise in Form von einem oder mehreren Quadrupolmagneten aufweist.

7. Strahlführungssystem nach einem der Ansprüche 1 bis 6,
wobei das Strahlführungssystem (2, 2') in Richtung (12) des Strahls geladener Teilchen (6) gesehen vor der ersten magnetischen Strahlablenkeinheit (30a) eine Kollimatoreinheit (24) aufweist.

8. Strahlführungssystem nach einem der Ansprüche 1 bis 7,
wobei das Strahlführungssystem (2, 2') mindestens zwei magnetische Strahlablenkeinheiten (30a, 30b, 30c, 30d) und zwischen zwei der mindestens zwei magnetischen Strahlablenkeinheiten (30b, 30c) eine Kollimatoreinheit (38) aufweist.

9. Strahlführungssystem nach einem der Ansprüche 1 bis 8,
wobei das Strahlführungssystem (2, 2') zumindest abschnittsweise (8) beweglich, insbesondere rotierbar ausgebildet ist.

10. Strahlführungssystem nach einem der Ansprüche 1 bis 9,
wobei das Strahlführungssystem (2, 2') eine Energiekorrektureinheit (18) zur Einstellung der Energie der geladenen Teilchen des Strahls geladener Teilchen (6) aufweist.

11. Strahlführungssystem nach Anspruch 10,
wobei die Energiekorrektureinheit (18) mindestens ein quer zum Strahl geladener Teilchen (6) verschiebbares blockförmiges Energiekorrekturelement (62a, 62b, 62c, 62d, 62e) und mindestens ein quer zum Strahl geladener Teilchen (6) verschiebbares keilförmiges Energiekorrekturelement (64a, 64b) aufweist.

12. Strahlführungssystem nach Anspruch 10 oder 11,
wobei die Energiekorrektureinheit (18) eine Mehrzahl von quer zum Strahl geladener Teilchen (6) verschiebbaren blockförmigen Energiekorrekturelementen (62a, 62b, 62c, 62d, 62e) aufweist, welche unterschiedliche Einstellungen der Energie der geladenen Teilchen des Strahls geladener Teilchen (6) ermöglichen.

13. Strahlführungssystem nach einem der Ansprüche 10 bis 12,
wobei die Energiekorrektureinheit (18) mehrere, insbesondere zwei quer zum Strahl geladener Teilchen verschiebbare keilförmige Energiekorrekturelemente (64a, 64b) aufweist.

14. Strahlführungssystem nach einem der Ansprüche 10 bis 13,
wobei die Energiekorrektureinheit (18) zumindest teilweise aus Borcarbid hergestellt ist.

15. Strahlführungssystem nach einem der Ansprüche 10 bis 14,
wobei das Strahlführungssystem (2,2') in Richtung (12) des Strahls geladener Teilchen (6) gesehen nach der Energiekorrektureinheit (18) eine Kollimatoreinheit (20) aufweist.

16. Strahlführungssystem nach einem der Ansprüche 10 bis 15
wobei das Strahlführungssystem (2, 2') zwischen der Energiekorrektureinheit (18) und der in Richtung (12) des Strahls geladener Teilchen (6) gesehen ersten magnetischen Strahlablenkeinheit (30a) eine Driftstrecke (22) aufweist, welche frei von magnetischen Strahlablenkeinheiten und/oder magnetischen Strahlformungseinheiten ist.

17. Teilchenstrahl-Therapieanlage (1)
- mit einer Strahlerzeugungseinheit (4) zur Erzeugung eines Strahls geladener Teilchen (6), insbesondere Ionen, vorzugsweise Protonen, und
- mit einem Strahlführungssystem (2, 2') nach einem der Ansprüche 1 bis 16, wobei vorzugsweise in Richtung (12) des Strahls geladener Teilchen (6) gesehen hinter der Strahlerzeugungseinheit eine Strahlformungseinheit 16 vorgesehen wird.

18. Verfahren umfassend die Schritte
- Erzeugen eines Strahls geladener Teilchen, insbesondere Ionen, vorzugsweise Protonen,
- Führen des Strahls geladener Teilchen mittels eines Strahlführungssystems nach einem der Ansprüche 1 bis 16.
